# EUROPÄISCHE PATENTSCHRIFT

(11) **EP 0 365 737 B1**
(45) Veröffentlichungstag und Bekanntmachung des Hinweises auf die Patenterteilung: **12.05.1993**
(21) Anmeldenummer: 89102330.1
(22) Anmeldetag: 10.02.1989
(51) Int. Cl.: A61B 6/00, A61B 6/04

(54) **Röntgenuntersuchungsgerät**
X-ray examination apparatus
Appareil d'examen par radiographie

(30) Priorität: 25.10.1988 EP 88117778
(43) Veröffentlichungstag der Anmeldung: 02.05.1990
(73) Patentinhaber: SIEMENS AKTIENGESELLSCHAFT, 80333 München (DE)
(72) Erfinder: Cramer, Bernhard Maximilian, Dr., D-5600 Wuppertal 2 (DE); Jenner, Erhard, Dipl.-Ing.(FH), D-8521 Hessdorf (DE); Minderle, Wolfgang, D-8530 Neustadt (DE)

(56) Entgegenhaltungen:
- DE-A- 2 356 276
- FR-A- 1 539 969
- FR-A- 2 247 954
- FR-A- 2 464 057

## Beschreibung

Die Erfindung betrifft ein Röntgenuntersuchungsgerät mit einem motorisch verstellbaren Bildaufnahmesystem und einer verstellbar gelagerten Patientenlagerungsvorrichtung.

Es ist ein Röntgenuntersuchungsgerät dieser Art bekannt, bei dem ein Patientenlagerungstisch einseitig auf einem Sockel zweidimensional verstellbar gelagert ist und von einem C-Bogen umgriffen wird, an dessen Enden ein Röntgenstrahler und ein Röntgenbildverstärker befestigt sind. Die Einstellung des Patienten relativ zum Bildaufnahmesystem erfolgt dabei sowohl durch Verstellung des Patientenlagerungstisches als auch des Bildaufnahmesystems. Die Einstellvorgänge sind dabei aber nicht miteinander koordiniert. Dies bedeutet, daß die Ausrichtung des Patienten in bezug auf das Bildaufnahmesystem durch individuelle Verstellung des Patientenlagerungstisches und des Bildaufnahmesystems selbst erfolgt.

In der FR-A-1 539 969 ist ein Röntgenuntersuchungsgerät beschrieben, bei dem ein Motor die Patientenlagerungsvorrichtung und das Bildaufnahmesystem gegenläufig zueinander verstellt. In der FR-A-2 464 057 ist ein radiologisches Untersuchungsgerät mit motorunterstützter, manueller Verstellung von zwei Wagen beschrieben. Schließlich ist durch die DE-A-2 356 276 ein Röntgenzielgerät bekannt, bei dessen Verstellung automatisch eine verstellbare Komponente ebenfalls verstellt wird. Die hier beschriebene Technik ist auf die Nachführung der Komponente an die Verstellbewegung des Zielgerätes ausgerichtet.

Der Erfindung liegt die Aufgabe zu Grunde, ein Röntgenuntersuchungsgerät der eingangs genannten Art so auszubilden, daß die Einstellung des Patienten in bezug auf das Bildaufnahmesystem gegenüber dem Stand der Technik vereinfacht ist und insbesondere schneller erfolgt.

Diese Aufgabe ist erfindungsgemäß dadurch gelöst, daß die Patientenlagerungsvorrichtung von Hand verstellbar ist und daß dem Bildaufnahmesystem und der Patientenlagerungsvorrichtung eine Steuervorrichtung zugeordnet ist, die so ausgebildet ist, daß bei einer manuellen Bewegung der Patientenlagerungsvorrichtung in einer Richtung eine automatische motorische Bewegung des Bildaufnahmesystems in der entgegengesetzten Richtung erfolgt. Bei der vorliegenden Erfindung sind die manuelle Verstellung der Patientenlagerungsvorrichtung und die motorische Verstellung des Bildaufnahmesystems derart miteinander koordiniert, daß bei einer Verschiebung der Patientenlagerungsvorrichtung in Längsrichtung eine gegenläufige motorische Verschiebung des Bildaufnahmesystems erfolgt, so daß auf diese Weise eine schnelle Einstellung des Patienten in bezug auf das Bildaufnahmesystem gewährleistet ist.

Eine Weiterbildung der Erfindung besteht darin, daß der gesamte Stellbereich der Patientenlagerungsvorrichtung in Teilbereiche unterteilt ist, denen Schaltmittel zugeordet sind, die eine gegenläufige Bewegung des Bildaufnahmesystems nur in vorbestimmten Teilbereichen bewirken. Dadurch kann z.B. erreicht werden, daß ein zentraler Bereich der Patientenlagerungsvorrichtung vorhanden ist, in dem keine automatische gegenläufige Verstellung des Bildaufnahmesystems erfolgt. Mit dem Überschreiten dieses Bereiches erfolgt dann eine gegenläufige Verstellung. Dabei ist es möglich, mehrere Geschwindigkeitsbereiche vorzusehen, die verschiedenen Teilbereichen zu beiden Seiten des zentralen Bereiches der Patientenlagerungsvorrichtung zugeordnet sind. Wird ein erster Teilbereich auf einer Seite des zentralen Bereiches erreicht, so hat beispielsweise die Verstellgeschwindigkeit für die gegenläufige Verstellung einen ersten, niedrigen Wert und beim Überschreiten dieses ersten Teilbereiches wird sie auf einen zweiten Wert erhöht.

Die Erfindung ist nachfolgend anhand der Zeichnung näher erläutert. Es zeigen:
- Fig. 1: ein Röntgenuntersuchungsgerät nach der Erfindung, und
- Fig. 2: eine Variante der Steuermittel des Röntgenuntersuchungsgerätes gemäß Fig. 1.

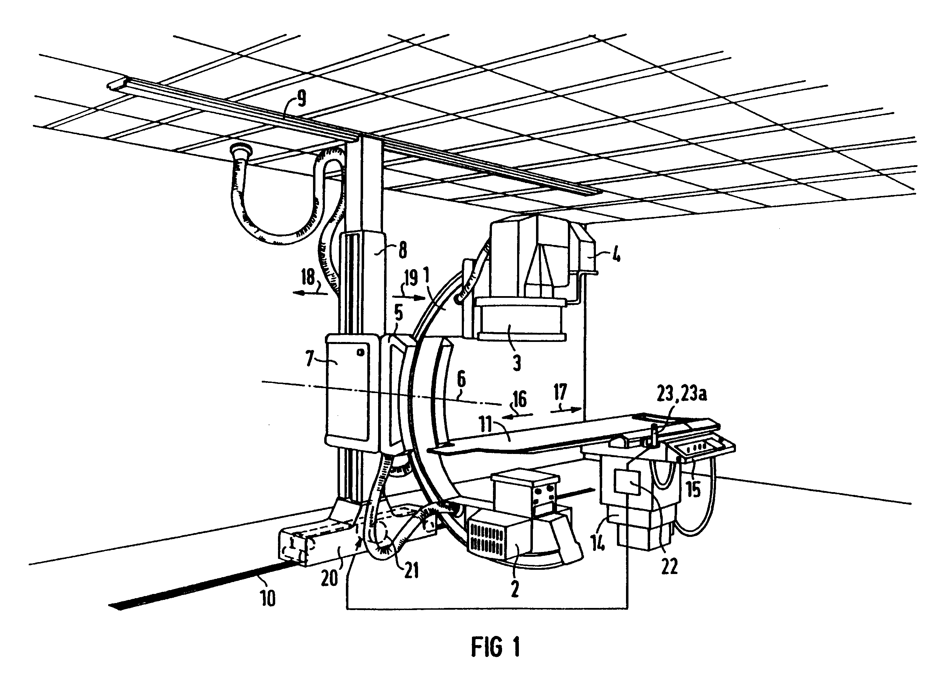

In der Fig. 1 ist ein C-Bogen 1 dargestellt, der an seinen Enden einen Röntgenstrahler 2 und einen Röntgenbildverstärker 3 mit nachgeschalteter Einzelbildkamera 4 sowie nicht sichtbarer Fernsehkamera trägt. Der C-Bogen 1 bildet zusammen mit den Komponenten 2, 3, 4 ein Bildaufnahmesystem und ist längs seines Umfanges an einem Halter 5 verstellbar gelagert, welcher um eine horizontale Achse 6 schwenkbar mit einem Wagen 7 verbunden ist. Der Wagen 7 ist an einer vertikalen Säule 8 längsverschiebbar gelagert, welche in einer Deckenschiene 9 und in einer Bodenschiene 10, die parallel zueinander und zur Längsrichtung eines Patientenlagerungstisches 11 verlaufen, verfahrbar ist. Der C-Bogen 1 umgreift den Patientenlagerungstisch 11 quer. Der Patientenlagerungstisch 11 ist auf einem Sockel 14 höhenverstellbar gelagert, an dem ein Bedienpult 15 befestigt ist. Er ist gegenüber dem Sockel 14 manuell in Längs- und Querrichtung verstellbar (schwimmende Lagerung).

Die Verstellung des Patientenlagerungstisches 11 in Längsrichtung ist durch die Pfeile 16, 17 angegeben. Die Pfeile 18, 19 geben die Verstellung der Säule 8 in den Schienen 9, 10 an.

Zur Verstellung der Säule 8 ist in ihrem Sockel 20 der Motor 21 vorgesehen. Der Motor 21 wird von einer Steuervorrichtung 22 angesteuert, an der ein Geber 23 im Handgriff 23a angeschlossen ist, der ein der Verstellung des Patientenlagerungstisches 11 in Richtung der Pfeile 16, 17 entsprechendes elektrisches Signal liefert.

Wird der Patientenlagerungstisch 11 von Hand in Richtung des Pfeiles 16 verschoben, so bewirkt die Steuervorrichtung 22 auf Grund des Signals des Gebers 23 über den Motor 21 eine automatische gegenläufige Verstellung der Säule 8 mit dem Bildaufnahmesystem 1, 2, 3, 4 in Richtung des Pfeiles 19. Wird der Patientenlagerungstisch 11 in Richtung des Pfeiles 17 verstellt, so erfolgt automatisch eine gegenläufige, motorische Verstellung der Säule 8 mit dem Bildaufnahmesystem 1, 2, 3, 4 in Richtung des Pfeiles 18. Die Einstellung eines auf dem Patientenlagerungstisch 11 liegenden Patienten in bezug auf das Bildaufnahmesystem 1, 2, 3, 4 kann auf Grund der beschriebenen automatischen, gegenläufigen Verstellung sehr schnell und einfach erfolgen.

In der Fig. 1 ist nur die gegenläufige Verstellung des Patientenlagerungstisches 11 sowie der Säule 8 mit dem Bildaufnahmesystem 1, 2, 3, 4 in einer Dimension dargestellt. Es ist jedoch auch möglich, eine derartige gegenläufige Verstellung auch in einer zweiten Dimension, die senkrecht zu ersten Dimension liegt, vorzusehen. Wird beispielsweise der Patientenlagerungstisch 11 in Richtung der Achse 6 in Richtung auf die Säule 8 bewegt, so kann dabei automatisch eine Verstellung des Bildaufnahmesystems 1, 2, 3, 4 in Richtung der Achse 6 von der Säule 8 weg erfolgen und umgekehrt. Hierzu kann der Halter 5 mit dem Wagen 7 in Richtung der Achse 6 motorisch verstellbar verbunden sein.

Die geschilderte automatische, gegenläufige Verstellung des Bildaufnahmesystems 1, 2, 3, 4 zu dem Patientenlagerungstisch 11 mit Hilfe einer Parallelverschiebung der Säule 8 zur Längsrichtung des Patientenlagerungstisches 11 erfolgt auf Grund des vom Geber 23 gelieferten, der Bewegungsrichtung des Patientenlagerungstisches 11 entsprechenden Signals.

Es ist auch möglich, für die Verstellung des Patientenlagerungstisches 11 ebenfalls einen Motor vorzusehen, welcher manuell steuerbar ist.

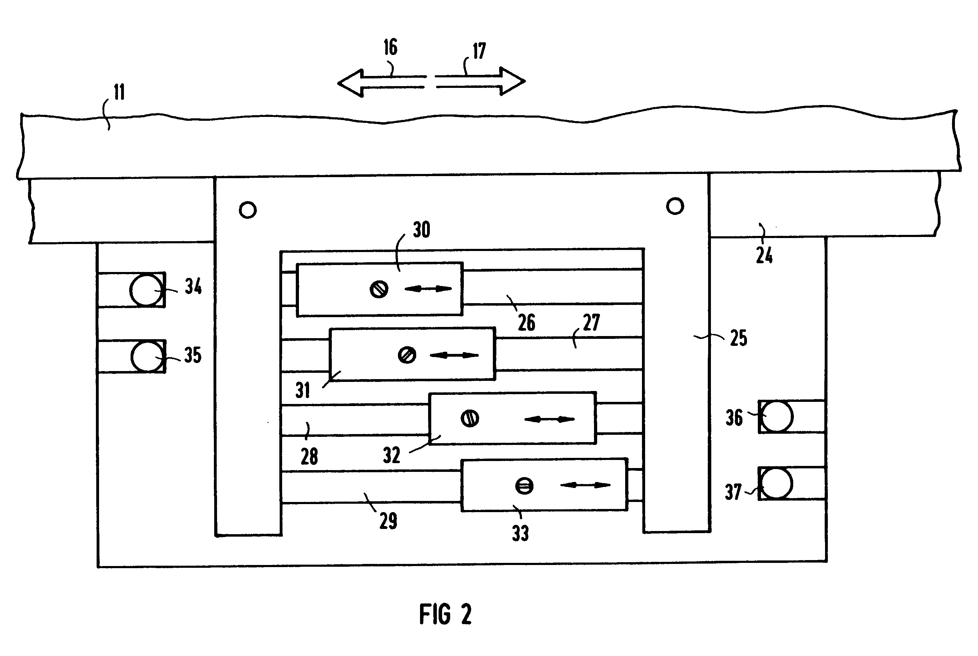

Die Fig. 2 zeigt, daß an der Zubehörschiene 24 des Patientenlagerungstisches 11 ein Halter 25 für vier parallele Führungsstangen 26, 27, 28, 29 angebracht ist. Auf den Führungsstangen 26 bis 29 sind verstellbare Betätigungsglieder 30 bis 33 angebracht, die mit Endschaltern 34 bis 37 zusammenwirken.

Wird der Patientenlagerungstisch 11 innerhalb eines zentralen Bereiches verstellt, innerhalb dessen keiner der Endschalter 34 bis 37 von den Betätigungsgliedern 30 bis 33 betätigt wird, so bleibt das Bildaufnahmesystem 1, 2, 3, 4 in Ruhe, wird also nicht gegenläufig zum Patientenlagerungstisch 11 verstellt. Wird der zentrale Stellbereich des Patientenlagerungstisches 11 überschritten, so betätigt auf der einen Seite das Betätigungsglied 30 den Endschalter 34 oder auf der anderen Seite das Betätigungsglied 33 den Endschalter 37. Dadurch wird die Steuervorrichtung 22 aktiviert. Beim überschreiten des zentralen Bereiches, d.h. bei betätigten Endschaltern 34 oder 37, erfolgt also eine gegenläufige Verstellung des Bildaufnahmesystems 1, 2, 3, 4, und zwar mit einer ersten niedrigen Geschwindigkeit.

Wird der Stellbereich für diese niedrige Geschwindigkeit überschritten, so betätigt das Betätigungsglied 31 den Endschalter 35 oder das Betätigungsglied 32 den Endschalter 36. Dadurch wird die Geschwindigkeit zur gegenläufigen Verstellung des Bildaufnahmesystems 1 bis 4 erhöht.

Der gesamte Stellbereich des Patientenlagerungstisches 11 ist also in fünf Teilbereiche (zentraler Bereich, an den sich zu beiden Seiten je zwei Teilbereiche anschließen) unterteilt, denen die Komponenten 30 bis 37 als Schaltmittel zugeordnet sind, die eine gegenläufige Bewegung des Bildaufnahmesystems 1, 2, 3, 4 nur in vorbestimmten Teilbereichen, nämlich in den zu beiden Seiten außerhalb des zentralen Bereiches liegenden Teilbereichen bewirken. Alle Teilbereiche sind durch Verstellung der Betätigungsglieder 30 bis 33 auf den Führungsstangen 26 bis 29 einstellbar. Die Steuervorrichtung 22 bewirkt durch Auswertung der Signale der Endschalter 34 bis 37, daß die Verstellgeschwindigkeit des Bildaufnahmesystems 1, 2, 3, 4 in Abhängigkeit vom Stellhub des Patientenlagerungstisches eingestellt wird.

## Patentansprüche

1. Röntgenuntersuchungsgerät mit einem motorisch verstellbaren Bildaufnahmesystem (1, 2, 3, 4) und einer verstellbar gelagerten Patientenlagerungsvorichtung (11), **dadurch gekennzeichnet,** daß die Patientenlagerungsvorrichtung (11) von Hand verstellbar ist und daß dem Bildaufnahmesystem (1, 2, 3, 4) eine Steuervorrichtung (22) zugeordnet ist, die so ausgebildet ist, daß bei einer manuellen Bewegung der Patientenlagerungsvorrichtung (11) in einer Richtung (16, 17) eine automatische motorische Bewegung des Bildaufnahmesystems (1, 2, 3, 4) in der entgegengesetzten Richtung erfolgt.

2. Röntgenuntersuchungsgerät nach Anspruch 1, **dadurch gekennzeichnet,** daß bei manueller Verstellung der Patientenlagerungsvorrichtung (11) der Steuervorrichtung (22) ein der Stellrichtung entsprechendes elektrisches Signal zugeführt wird, auf Grund dessen die Steuerung der Verstellung des Bildaufnahmesystems (1, 2, 3, 4) erfolgt.

3. Röntgenuntersuchungsgerät nach Anspruch 2, **dadurch gekennzeichnet,** daß das Bildaufnahmesystem (1, 2, 3, 4) an einer parallel zur Längsrichtung der Patientenlagerungsvorrichtung (11) motorisch verstellbar gelagerten Säule (8) angeordnet ist.

4. Röntgenuntersuchungsgerät nach einem der Ansprüche 1 bis 3, **dadurch gekennzeichnet,** daß der gesamte Stellbereich der Patientenlagerungsvorrichtung (11) in Teilbereiche unterteilt ist, denen Schaltmittel (30 bis 37) zugeordnet sind, die eine gegenläufige Bewegung des Bildaufnahmesystems (1, 2, 3, 4) nur in vorbestimmten Teilbereichen bewirken.

5. Röntgenuntersuchungsgerät nach Anspruch 4, **dadurch gekennzeichnet,** daß ein zentraler Teilbereich der Patientenlagerungsvorrichtung (11) vorhanden ist, innerhalb dessen keine gegenläufige Verstellung des Bildaufnahmesystems (1, 2, 3, 4) erfolgt und daß sich zu beiden Seiten des zentralen Teilbereiches Teilbereiche anschließen, innerhalb deren die gegenläufige Verstellung des Bildaufnahmesystems (1, 2, 3, 4) erfolgt.

6. Röntgenuntersuchungsgerät nach Anspruch 4 oder 5, **dadurch gekennzeichnet,** daß die Teilbereiche einstellbar sind.

7. Röntgenuntersuchungsgerät nach einem der Ansprüche 1 bis 6, **dadurch gekennzeichnet,** daß die Steuervorrichtung (22) so ausgebildet ist, daß sie in Abhängigkeit vom Stellhub der Patientenlagerungsvorrichtung (11) die Verstellgeschwindigkeit des automatisch gegenläufig verstellten Bildaufnahmesystems (1, 2, 3, 4) einstellt.

## Claims

1. X-ray examination device having a motor-driven adjustable imaging system (1, 2, 3, 4) and an adjustably mounted patient support device (11), characterised in that the patient support device (11) is manually adjustable, and in that a control device (22) is associated with the imaging system (1, 2, 3, 4) and is constructed in such a way that when there is manual movement of the patient support device (11) in one direction (16, 17), an automatic motor-driven movement of the imaging system (1, 2, 3, 4) occurs in the opposite direction.

2. X-ray examination device according to claim 1, characterised in that when there is manual adjustment of the patient support device (11) an electrical signal, corresponding to the direction of adjustment, is fed to the control device (22), and hence control of the adjustment of the imaging system (1, 2, 3, 4) takes place.

3. X-ray examination device according to claim 2, characterised in that the imaging system (1, 2, 3, 4) is arranged on a column (8) adjustably mounted by motor parallel to the longitudinal direction of the patient support device (11).

4. X-ray examination device according to one of claims 1 to 3, characterised in that the entire adjustment range of the patient support device (11) is divided into sub-ranges with which switch means (30 to 37) are associated which cause an oppositely directed movement of the imaging system (1, 2, 3, 4) only in predetermined sub-ranges.

5. X-ray examination device according to claim 4, characterised in that there is a central sub-range of the patient support device (11) within which no oppositely directed adjustment of the imaging system (1, 2, 3, 4) takes place, and in that there are sub-ranges adjacent to both sides of the central sub-range, within which oppositely directed adjustment of the imaging system (1, 2, 3, 4) takes place.

6. X-ray examination device according to claim 4 or 5, characterised in that the sub-ranges are adjustable.

7. X-ray examination device according to one of claims 1 to 6, characterised in that the control device (22) is constructed so that it sets the adjustment speed of the automatically oppositely adjusted imaging system (1, 2, 3, 4) in dependence on the displacement of the patient support device (11).

## Revendications

1. Appareil de radiodiagnostic comportant un système d'enregistrement d'images (1,2,3,4), déplaçable au moyen d'un moteur, et un dispositif formant couchette pour patient (11), monté de manière à être réglable, caractérisé par le fait que le dispositif formant couchette pour patient (11) est réglable manuellement et qu'au système d'enregistrement d'images (1,2,3,4) est associé un dispositif de commande (22), agencé de telle sorte que, lors d'un déplacement manuel du dispositif formant couchette pour patient (11) dans une direction (16,17), il se produit un déplacement automatique, déclenché par un moteur, du système d'enregistrement d'images (1,2,3,4) dans la direction opposée.

2. Appareil de radiodiagnostic suivant la revendication 1, caractérisé par le fait que dans le cas d'un réglage manuel du dispositif formant couchette pour patient (11), au dispositif de commande (22) est envoyé un signal électrique, qui correspond au sens de réglage et sur la base duquel s'effectue la commande du réglage du système d'enregistrement d'images (1,2,3,4).

3. Appareil de radiodiagnostic suivant la revendication 2, caractérisé par le fait que le système d'enregistrement d'images (1,2,3,4) est disposé sur une colonne (8) qui est montée de manière à être déplaçable par un moteur parallèlement à la direction longitudinale du dispositif formant couchette pour patient (11).

4. Appareil de radiodiagnostic suivant l'une des revendications 1 à 3, caractérisé par le fait que l'ensemble de la plage de réglage du dispositif formant couchette pour patient (11) est subdivisé en des zones partielles, auxquelles sont associés des moyens de commutation (30 à 37), qui déclenchent un déplacement en sens opposé du système d'enregistrement d'images (1,2,3,4), uniquement dans des zones partielles prédéterminées.

5. Appareil de radiodiagnostic suivant la revendication 4, caractérisé par le fait qu'il est prévu une zone centrale partielle du dispositif formant couchette pour patient (11), à l'intérieur de laquelle il ne se produit aucun déplacement en sens opposé du système d'enregistrement d'images (1,2,3,4) et qu'aux deux côtés de la zone centrale partielle se raccordent des zones partielles, à l'intérieur desquelles s'effectue le déplacement en sens opposé du système d'enregistrement d'images (1,2,3,4).

6. Appareil de radiodiagnostic suivant l'une des revendications 3 ou 4, caractérisé par le fait que les zones partielles sont réglables.

7. Appareil de radiodiagnostic suivant l'une des revendications 1 à 6, caractérisé par le fait que le dispositif de commande (22) est agencé de telle sorte qu'il règle la vitesse de déplacement du système d'enregistrement d'images (1,2,3,4), qui est déplacé automatiquement en sens opposé, en fonction de la course de réglage du dispositif formant couchette pour patient (11).
